# DEMANDE DE BREVET EUROPEEN

(11) **EP 4 391 572 A1**
(43) Date de publication de la demande: **26.06.2024**
(21) Numéro de dépôt: 23217843.4
(22) Date de dépôt: 19.12.2023
(51) Int. Cl.: H04N 25/30, G02B 6/10, G01T 1/20, H01L 27/146, F21V 8/00

(54) **DETECTEUR NUMERIQUE POSSEDANT UN GENERATEUR DE LUMIERE PERMETTANT UN EFFACEMENT OPTIQUE**

(30) Priorité: 21.12.2022 FR 2214164
(71) Demandeur: Trixell, 38430 Moirans (FR)
(72) Inventeur: BACHER, Guillaume, 38430 MOIRANS (FR); ZEKHNINI, Mohamed, 38430 MOIRANS (FR); CIARAMELLA, Frédéric, 38430 MOIRANS (FR); CHARRASSE, Patrick, 38430 MOIRANS (FR); MAZEL, Frédéric, 38430 MOIRANS (FR)
(74) Mandataire: Atout PI Laplace

(57) **Abrégé**

L'invention concerne un détecteur numérique (10) à l'état solide d'un rayonnement incident (100), le détecteur (10) comprenant un capteur photosensible (30) et un générateur de lumière (40), le capteur photosensible (30) comprenant des éléments photosensibles (31) organisés en matrice, le générateur de lumière (40) étant destiné à effacer optiquement les éléments photosensibles (31), où le générateur de lumière (40) comprend :
- un guide de lumière (42) comprenant une face avant tournée vers l'avant du détecteur (10), une face arrière opposée à la face avant et au moins une face latérale s'étendant entre la face avant et la face arrière, et
- au moins une source de lumière (41) disposée en périphérie latérale du guide de lumière (42) et où le générateur de lumière (40) est configuré pour que ladite au moins une source de lumière (41) injecte de la lumière au travers d'au moins une des faces du guide de lumière (42), ledit guide de lumière (42) étant configuré pour répartir la lumière provenant de ladite au moins une source de lumière (41) sur toute la matrice d'éléments photosensibles (31).

## Description

La présente invention concerne un détecteur de rayonnement X à l'état solide comprenant un capteur photosensible pouvant être associé à un convertisseur de rayonnement.

De tels détecteurs de rayonnement sont par exemple décrits dans le brevet français FR 2 803 081 dans lequel un capteur formé de photodiodes en silicium amorphe est associé à un convertisseur de rayonnement.

Le capteur photosensible est généralement réalisé à partir d'éléments photosensibles à l'état solide arrangés en matrice. Les éléments photosensibles sont réalisés à partir de matériaux semi-conducteurs, le plus souvent du silicium monocristallin pour les capteurs de type CCD ou CMOS, du silicium poly cristallin ou amorphe. Un élément photosensible comporte au moins une photodiode, un phototransistor ou une photo résistance. Ces éléments sont déposés sur un substrat, généralement une dalle de verre.

Ces éléments ne sont pas généralement pas sensibles directement aux rayonnements de longueurs d'ondes très courtes comme le sont les rayons X ou gamma. C'est pourquoi on associe le capteur photosensible à un convertisseur de rayonnement, aussi appelé écran scintillateur, qui comporte une couche d'une substance scintillatrice. Cette substance a la propriété, lorsqu'elle est excitée par de tels rayonnements, d'émettre un rayonnement de longueur d'onde supérieure, par exemple de la lumière visible ou proche du visible, auquel est sensible le capteur. La lumière émise par le convertisseur de rayonnement illumine les éléments photosensibles du capteur qui effectuent une conversion photoélectrique et délivrent des signaux électriques exploitables par des circuits appropriés.

Afin d'améliorer la qualité des images obtenues, il est connu de procéder en amont à une prise d'image à une étape dite « d'effacement » au cours de laquelle les éléments photosensibles sont effacés optiquement au moyen d'un flash de lumière uniformément réparti sur l'ensemble des éléments photosensibles.

A ce jour le flash de lumière est obtenu au moyen d'un générateur de lumière formé d'une matrice de diodes électroluminescentes placée sur la face arrière du détecteur. Par convention, on appelle face avant du détecteur la face exposée au rayonnement X et face arrière la face opposée à la face avant. Lors du flash de lumière, la matrice de diodes électroluminescentes émet un rayonnement visible qui traverse la dalle de verre formant le substrat du capteur photosensible puis se réfléchit sur des faces situées en amont du capteur photosensible avant d'atteindre les éléments photosensibles.

Dans certaines applications en imagerie médicale où le détecteur est en mouvement durant un examen, comme par exemple en tomodensitométrie, une lame d'air présente entre le générateur de lumière et le détecteur peut voir son épaisseur évoluer avec les mouvements du détecteur. La variation de cette lame d'air peut entraîner la formation d'une image fantôme du générateur de lumière sur les images issues du détecteur. Un autre inconvénient des générateurs de lumière réalisés au moyen de diodes électroluminescentes ou de lampes réside dans l'épaisseur de tels générateurs. On a également tenté de placer en face arrière du détecteur une couche de diodes électroluminescentes organiques appelées par la suite OLED en référence à leur dénomination anglo-saxonne : « Organic Light- Emitting Diode ». Ce type de diode réalisée sous forme d'une couche de matériau luminescent disposé entre deux électrodes dont une au moins est transparente pour permettre l'émission de lumière hors de la couche. Pour les OLED, Il est connu d'utiliser une électrode transparente réalisée en oxyde d'indium dopé à l'étain appelé par la suite ITO en référence à sa dénomination anglo-saxonne : « Indium tin oxide ». Ce type d'électrode est particulièrement adapté pour des couches d'OLED de petites surfaces. En effet, on s'est rendu compte que l'ITO présente une résistivité importante. Pour une couche d'OLED de grande surface, la luminosité n'est pas homogène. L'éclairement est plus important sur les bords qu'au centre de la couche. Les détecteurs de rayonnement X sont obligatoirement de grande dimension puisqu'il est pratiquement impossible de focaliser ce type de rayonnement du fait de son énergie élevée. Dans la radiologie médicale, on a réalisé des détecteurs numériques dont les dimensions sont semblables à celles des films argentiques utilisés par le passé. On trouve couramment des détecteurs dont les dimensions dépassent 400 mm de côté. Pour de telles dimensions, une couche d'OLED possédant une électrode d'ITO ne permettrait pas d'être utilisée pour réaliser un flash de lumière homogène. En outre les valeurs importantes de constante de temps liées à la résistance élevées et à la grande capacité à charger/décharger de l'OLED entrainent des temps de réponse lents, incompatibles avec l'obtention de flash lumineux brefs à une cadence élevée.

L'invention vise à pallier tout ou partie des problèmes cités plus haut en proposant un détecteur numérique de rayonnement à l'état solide dans lequel un flash de lumière uniformément réparti permet la réinitialisation des pixels. L'invention présente un intérêt important pour un détecteur de rayonnement X. Il est néanmoins possible de mettre en oeuvre l'invention pour d'autres types de rayonnements comme par exemple les rayonnements gamma.

A cet effet l'invention concerne un détecteur numérique à l'état solide d'un rayonnement incident, le détecteur comprenant un capteur photosensible et un générateur de lumière, le capteur photosensible comprenant des éléments photosensibles organisés en matrice, le générateur de lumière étant destiné à effacer optiquement les éléments photosensibles où le générateur de lumière comprend :
- un guide de lumière comprenant une face avant tournée vers l'avant du détecteur une face arrière opposée à la face avant et au moins une face latérale s'étendant entre la face avant et la face arrière, et
- au moins une source de lumière disposée en périphérie latérale du guide de lumière et où le générateur de lumière est configuré pour que ladite au moins une source de lumière injecte de la lumière au travers d'au moins une des faces du guide de lumière
ledit guide de lumière étant configuré pour répartir la lumière provenant de ladite au moins une source de lumière sur toute la matrice d'éléments photosensibles.

La configuration du détecteur numérique de l'invention permet avantageusement de limiter l'encombrement du générateur de lumière. Ainsi ladite au moins une source de lumière est disposée en regard ou à proximité des faces latérales du guide de lumière. Dans la mesure où la lumière arrive de manière latérale dans le guide, la distance parcourue pour atteindre les éléments photosensibles est allongée, ce qui améliore l'aspect diffus de la lumière au niveau des éléments photosensible et baisse, voire annule, la distinctivité de la lumière provenant de chaque source de lumière. L'invention permet également un fonctionnement du détecteur à des fréquences élevées et des impulsions lumineuses brèves avec des temps d'allumage et d'extinction courts.

L'invention permet également de réduire à la fois le nombre de sources de lumière et à la fois la taille des circuits imprimés associés, comparativement à l'art antérieur. En outre, dans la mesure où la surface des circuits imprimés est réduite, il est obtenu une forte réduction, voire une absence, de déformation de ces derniers et donc une diminution des artefacts dans les images. Cette diminution de taille de surface permet également de réduire la probabilité de choc en cas de chute du détecteur numérique. Le remplacement des sources lumineuses est également facilité.

Dans un mode de réalisation de l'invention, le guide de lumière comprend une texturation diffusante afin d'obtenir une répartition uniforme de la lumière au niveau de la matrice d'éléments photosensibles.

Selon un mode de réalisation de l'invention, le guide de lumière comprend un ensemble de microbulles judicieusement disposées en son sein afin d'obtenir une répartition uniforme de la lumière au niveau de la matrice d'éléments photosensibles.

Selon un mode de réalisation de l'invention, le capteur photosensible comprend un substrat transparent ou translucide sur lequel est disposée la matrice d'éléments photosensibles et où la face avant du guide de lumière est disposée contre ledit substrat.

Notamment, le guide de lumière est fixé au substrat du capteur photosensible de préférence par collage.

Selon un mode de réalisation de l'invention, le capteur photosensible comprend un substrat transparent ou translucide sur lequel est disposée la matrice d'éléments photosensibles et où ledit substrat forme le guide de lumière.

Selon un mode de réalisation de l'invention, ladite au moins une source de lumière est disposée en regard d'une au moins des faces latérales du guide de lumière.

Selon un mode de réalisation de l'invention, le générateur de lumière comprend au moins un organe de réflexion apte à dévier la lumière provenant de ladite au moins une source de lumière vers le guide de lumière.

Notamment, l'organe de réflexion comprend au moins un dioptre et/ou au moins un réflecteur disposé en regard d'une des faces du guide de lumière.

Selon un mode de réalisation de l'invention, le guide de lumière comprenant au moins deux faces latérales opposées entre elles, dans lequel le générateur de lumière comprend au moins un réflecteur latéral disposé contre au moins une des faces latérales du guide de lumière de préférence ladite au moins une source de lumière est disposée en regard de la face latérale opposée à celle du réflecteur latéral.

Selon un mode de réalisation de l'invention, la matrice d'éléments photosensibles est disposée sur un substrat transparent ou translucide, où le guide de lumière est disposé en aval dudit substrat et comprend une partie latérale déportée par rapport à celle dudit substrat où le générateur de lumière comprend au moins un réflecteur supérieur s'étendant contre tout ou partie de la face supérieure de ladite au moins une partie latérale déportée du guide de lumière et où ladite au moins une source de lumière est disposée en périphérie de ladite au moins une partie latérale déportée du guide de lumière.

### Brève description des figures

L'invention sera mieux comprise à la lecture de la description qui va suivre donnée uniquement à titre d'exemple et faite en se référant aux dessins annexés dans lesquels :
La [Fig. 1] représente cinq configurations (a à e) d'un détecteur numérique selon l'invention. Chaque configuration est représentée selon une vue en coupe.
La [Fig.2] représente une vue en coupe du détecteur numérique de la figure 1a selon une première configuration d'un générateur de lumière. Dans la figure 2A, le générateur de lumière comprend une source de lumière et dans la figure 2B deux sources de lumière opposées entre elles.
La [Fig.3] représente une vue en coupe du détecteur numérique de la figure 1a selon une deuxième configuration d'un générateur de lumière. Dans la figure 3A, le générateur de lumière comprend une source de lumière et dans la figure 3B deux sources de lumière opposées entre elles.
La [Fig.4] représente une vue en coupe du détecteur numérique de la figure 1a selon une troisième configuration d'un générateur de lumière. Dans la figure 4A, le générateur de lumière comprend une source de lumière et dans la figure 4B deux sources de lumière opposées entre elles.

### Description détaillée de l'invention

Les réalisations suivantes sont des exemples. Bien que la description se réfère à un ou plusieurs modes de réalisations, ceci ne signifie pas nécessairement que chaque référence concerne le même mode de réalisation, ou que les caractéristiques s'appliquent seulement à un seul mode de réalisation. De simplement caractéristiques de différents modes de réalisation peuvent également être combinées et/ou inter-changées pour fournir d'autres modes de réalisation.

Dans la présente description, on peut indexer certains éléments ou paramètres, comme par exemple un premier réflecteur ou un deuxième réflecteur etc. Dans ce cas il s'agit d'un simple indexage pour différencier et nommer des éléments, paramètres ou critères proches, mais non identiques. Cette indexation n'implique pas une priorité d'un élément, paramètre ou critère par rapport à un autre et on peut aisément inter-changer de telles dénominations sans sortir du cadre de la présente description. Cette indexation n'implique pas non plus un ordre dans le temps.

Un détecteur numérique 1 à l'état solide selon l'invention permet de former une image fonction d'un rayonnement incident auquel il est sensible comme par exemple un rayonnement X 100. Le détecteur 10 comprend un écran scintillateur 20 permettant de convertir le rayonnement X 100 en rayonnement visible, un capteur photosensible 30 comprenant des éléments photosensibles 31 permettant de convertir le rayonnement visible issu de l'écran scintillateur 20 en signaux électriques formant l'image. Le détecteur comprend en sus un générateur de lumière 40 destiné à effacer optiquement les éléments photosensibles 31 du capteur 20.

Il est bien entendu que le détecteur 10 peut comprendre d'autres composants.

L'invention peut être mise en oeuvre dans un détecteur numérique 1 sans écran scintillateur et dont le capteur 30 effectue une conversion directe du rayonnement 100 en signal électrique. A cet effet, le capteur photosensible 30 comprend un photoconducteur directement sensible au rayonnement X 100 incident.

Plus précisément, lors du fonctionnement du détecteur 10, pour obtenir une image, on réalise une suite d'opérations formée d'une phase de prise d'image pendant laquelle le détecteur 10 est soumis au rayonnement X 100, suivie d'une phase de lecture au cours de laquelle on lit les signaux électriques issus de chacun des éléments photosensibles 31 puis d'une phase d'effacement optique au cours de laquelle on éclaire l'ensemble des éléments photosensibles 31 de façon uniforme à l'aide du générateur de lumière 40 et enfin une phase de réinitialisation électrique de l'ensemble des éléments photosensibles 31.

Par convention, on définit la face avant du détecteur 10 comme la face recevant en premier le rayonnement X 100, la face arrière étant opposée à la face avant. On définit également les positions relatives des différents composants du détecteur 10 entre eux par rapport au sens de propagation du rayonnement X. On dira par exemple que l'écran scintillateur 20 est situé en amont du capteur photosensible 30 car l'écran scintillateur 20 reçoit le rayonnement X 100 avant le capteur photosensible 30. Dans la pratique, le capteur 30 ne reçoit que très peu de rayonnement X 100, car le rayonnement X 100 est en quasi-totalité converti en rayonnement visible par l'écran scintillateur 20. La notion d'amont et d'aval peut également s'entendre pour le sens de propagation du rayonnement visible que l'écran scintillateur 20 émet en direction du capteur 20. Dans le cadre de l'invention, les termes « supérieur », « dessus », « amont » et « avant » seront pris pour synonymes, et les termes « inférieur », « aval », « dessous » et « arrière » seront pris pour synonymes.

L'écran scintillateur 20 est notamment au contact des éléments photosensibles 31. L'écran scintillateur 20 comprend notamment une substance scintillatrice 21 de la famille des halogénures alcalins ou des oxysulfures de terres rares. Notamment la substance scintillatrice 21 est composée de cristaux fluorescents tels que des cristaux d'iodure de sodium ou encore des cristaux d'iodure de césium dopé au sodium ou au thallium.

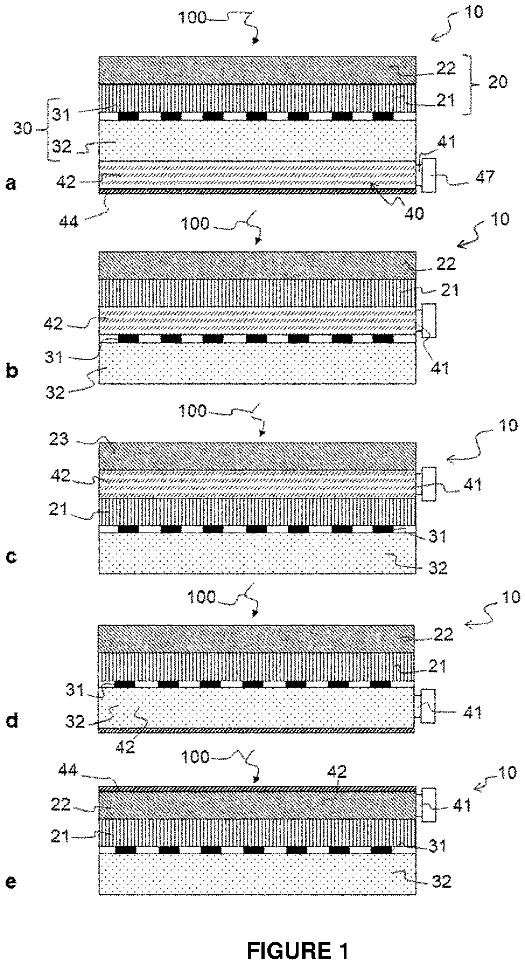

Dans une première configuration, dite de l'écran scintillateur rapporté, la substance scintillatrice 21 est déposée sur un support 22 que le rayonnement X 100 doit traverser avant d'atteindre le capteur 30. L'ensemble est alors collé sur le capteur 30 (figure 1).

Dans une deuxième configuration, dite du dépôt direct, le capteur 30 sert de support à la substance scintillatrice 21 qui est alors en contact direct et intime avec le capteur 30. La substance scintillatrice 21 est ensuite recouverte d'une feuille de protection 23.

Dans un mode de réalisation, le support 22 ou la feuille de protection 23 est notamment transparent ou translucide. Par transparent, il est entendu un élément qui permet de distinguer les objets nettement à travers son épaisseur. On peut considérer qu'un élément est transparent lorsqu'il présente une transmission lumineuse comprise entre 80% et 99%. Par translucide, il est entendu un élément qui se laisse traverser par la lumière dans le domaine du visible, mais sans permettre de distinguer nettement les objets. On peut considérer qu'un élément est translucide lorsqu'il présente une transmission lumineuse supérieure ou égale à 50% et inférieure à 80%.

Afin d'être transparent ou translucide, le support 22 ou la feuille de protection 23 est notamment en verre ou en matériau plastique. À titre d'exemple, des matériaux plastique présentant une bonne transparence et pouvant convenir à cet usage sont : le polypropylène, le polyéthylène téréphtalate, le polyméthacrylate de méthyle, le polycarbonate, le polytéréphtalate d'éthylène, l'acrylonitrile styrène acrylate, l'acrylonitrile butadiène styrène, ou encore les polyoléfines amorphes tels que les cyclo-oléfines copolymères (COC) ou les cyclo-oléfines-polymères (COP). Chacun de ces matériaux possède bien évidemment un indice de réfraction connu qui lui est propre. Concernant chacun de ces matériaux, et plus particulièrement le polypropylène, l'homme du métier sait en déterminer les membres correspondant aux matériaux transparents tels que définis plus haut.

De manière alternative, le support 22 ou la feuille de protection 23 est opaque et peut servir de réflecteur pour le renvoi du flux lumineux provenant du générateur de lumière 40 en direction des éléments photosensibles 31. En particulier, le support 22 ou la feuille de protection 23 peut être en aluminium ou en titane.

Le support 22 ou la feuille de protection 23 peut comprendre notamment une texturation diffusante au niveau de leur surface inférieure afin d'obtenir une répartition uniforme de la lumière au niveau de la matrice d'éléments photosensibles 31. Ladite texturation peut correspondre à un aspect dépoli de ladite surface, à une indentation ou à des points de sérigraphie. Ladite texturation peut notamment être obtenue par une attaque chimique contrôlée, par un usinage mécanique ou toute autre technique connue de l'homme du métier telle qu'un dépôt localisé ou une texturation laser permettant d'obtenir les caractéristiques de surface désirées. Notamment encore, le support 22 ou la feuille de protection 23 peut comprendre un ensemble de microbulles judicieusement disposées en son sein afin de permettre d'obtenir une diffusion de la lumière incidente. Notamment encore, la surface inférieure du support 22 ou de la feuille de protection 23 peut comprendre un ensemble de tâches dont les dimensions et/ou le nombre augmentent avec la distance depuis l'au moins une source de lumière 41 du générateur 40. En particulier les tâches sont circulaires. Ces aspects de l'invention sont notamment intéressants lorsque le guide de lumière 42 du générateur 40 est disposé en avant du support 22 ou de la feuille de protection 23, ou bien que ces derniers forment le guide de lumière 42 comme il sera vu en détail plus loin.

Les éléments photosensibles 31 sont organisés en matrice et sont notamment disposés sur un substrat 32. Les éléments photosensibles 31 Les éléments photosensibles 31 peuvent être sont réalisés à partir de matériaux semi-conducteurs, ils sont notamment en silicium monocristallin ou silicium poly cristallin ou amorphe, notamment en silicium amorphe hydrogéné. Les éléments photosensibles 31 peuvent être espacés les uns des autres sur le substrat 32 par des interstices. Dans les interstices les séparant peuvent être disposés des composants de fonction permettant le fonctionnement des éléments photosensibles 31. Les composants de fonction sont notamment disposés sur le substrat 32 également. On trouve par exemple un transistor suiveur, un transistor de lecture et un transistor de remise à zéro associés à chacun des éléments photosensibles 31. On désigne par pixel l'ensemble formé par un élément photosensible 31 et les composants de fonction associés. Dans les interstices peuvent également circuler des conducteurs électriques permettant d'alimenter, de commander et de lire les différents éléments photosensibles 31 au travers des composants de fonction.

Les éléments photosensibles 31 sont répartis sur tout ou partie de la surface supérieure du substrat 32. Pour la suite de la description, la région de la surface supérieure du substrat 32 comprenant les éléments photosensibles sera appelée « zone d'image ».

Le substrat 32 est notamment transparent ou translucide et a notamment une composition choisie parmi celles énoncées ci-dessus pour le support 22.

La surface inférieure du substrat 32 peut notamment comprendre une texturation diffusante et/ou un ensemble de tâches telles que décrites en relation avec le support 22. Le substrat 32 peut également comprendre un ensemble de microbulles en son sein, tel que décrit ci-dessus pour le support 22. Ces aspects de l'invention sont notamment intéressants lorsque le guide de lumière 42 du générateur 40 est disposé en arrière du substrat 32, ou bien que le substrat 32 sert de guide de lumière comme il sera vu en détail plus loin.

Le générateur de lumière 40 comprend au moins une source de lumière 41 émettant un flux de lumière visible, proche ultraviolet ou proche infrarouge dans un guide de lumière 42 configuré pour répartir la lumière provenant de ladite au moins une source de lumière 41 sur tous les éléments photosensibles 31. Le guide de lumière 42 comprend une face avant tournée vers l'avant du détecteur, une face arrière opposée à la face avant et au moins une face latérale s'étendant entre la face avant et la face arrière. Par « lumière proche ultraviolet » et « lumière proche infrarouge », il est entendu dans l'invention des longueurs d'ondes électromagnétiques dans l'ultraviolet ou dans l'infrarouge respectivement qui soient proches de la fenêtre visible de sorte que les éléments photosensibles 31 y soient sensibles. Notamment, l'au moins une source de lumière émet dans une gamme de longueur d'onde allant de 0,2 nm à 1 nm.

Ladite au moins une source de lumière 41 est disposée au niveau de la périphérie latérale du guide de lumière, c'est-à-dire au contact ou à proximité d'au moins une des faces latérales dudit guide de lumière 42. Cet agencement permet d'obtenir une optimisation de l'encombrement du générateur de lumière 40 par rapport à l'épaisseur du détecteur 10. Par « à proximité », il est entendu dans l'invention que ladite au moins une source de lumière 41 est disposée à une distance proche d'une face latérale du guide 42 lui permettant de diffuser un flux de lumière dans le guide de lumière 42. Ainsi, dans l'invention, l'au moins une source de lumière 41 n'est pas nécessairement disposée en regard d'une face latérale du guide de lumière 42, elle peut être disposée au-dessus ou au-dessous.

L'au moins une source de lumière 41 est disposée en périphérie de tout ou partie d'au moins une face latérale du guide de lumière 42. Notamment, l'ensemble des sources de lumière 41 sont disposées en périphérie le long d'une même face latérale du guide de lumière 42, ou le long de deux faces latérales opposées du guide de lumière 42.

Ladite au moins une source de lumière 41 est notamment une diode électroluminescente. Ladite au moins une source de lumière 41 peut être associée à une carte de circuit imprimé 47 gérant son allumage et son extinction.

Le générateur de lumière 40 peut notamment comprendre en outre au moins un organe de réflexion 43 apte à dévier la lumière provenant de ladite au moins une source de lumière 41 vers le guide de lumière 42. Ainsi, la lumière provenant de ladite au moins une source de lumière 41 peut être concentrée dans le guide de lumière, renvoyée dans le guide de lumière ou acheminée vers le guide de lumière 42, comme il sera vu en détail plus loin. Ce dernier aspect est en particulier intéressant lorsque ladite au moins une source de lumière 41 est disposée à proximité d'au moins une des faces latérales du guide de lumière 42, sans en être en regard.

L'au moins un organe de réflexion 43 comprend notamment au moins un dioptre et/ou au moins un réflecteur disposé en regard d'une des faces du guide de lumière 42. L'organe de réflexion 43 peut notamment comprendre :
- au moins un dioptre/réflecteur inférieur 44 disposé en regard de la surface inférieure du guide 42 et/ou
- au moins un dioptre/réflecteur supérieur 45 disposé en regard de la face supérieure du guide 42 et/ou
- au moins un dioptre/réflecteur latéral 46 disposé en regard de la ou d'une des faces latérales du guide 42.

La surface du dioptre ou du réflecteur 44, 45, 46 peut être plane et/ou suivre la forme de la surface du guide 42 disposée en regard. Alternativement, la surface du dioptre ou du réflecteur 44, 45, 46 peut être oblique à la surface dont il est en regard, cette surface correspondant notamment à la surface d'entrée. En particulier, le dioptre ou réflecteur oblique 44, 45, 46 présente notamment un angle avec la surface du guide 42 dont il est en regard de 45°.Le dioptre ou le réflecteur 44, 45, 46 est notamment au contact de la surface du guide 42 disposée en regard. Le dioptre ou réflecteur latéral 46 s'étend notamment également en regard d'une face latérale du capteur 30, notamment du substrat 32, et/ou d'une face latérale de l'écran scintillateur 20, notamment de la substance scintillatrice 21. Le dioptre ou le réflecteur supérieur 45 est notamment disposée au niveau d'une partie latérale déportée du guide de lumière 42, décrite en détail ci-après. Lorsque le dioptre ou réflecteur 44, 45, 46 est oblique, il permet notamment de diriger le flux lumineux de la au moins une source lumineuse 41 lorsque cette dernière n'est pas disposée en regard d'une face latérale du guide 42.

Le réflecteur 44, 45, 46 présente notamment une surface de réflexion de couleur blanche permettant d'obtenir une réflexion maximale. Le réflecteur 44, 45, 46 peut également être un miroir.

Le générateur de lumière 40 peut être configuré pour qu'au moins une partie du flux de lumière visible arrive de manière transversale au guide 42, notamment à l'aide dudit au moins un organe de de réflexion 43, de sorte à améliorer la diffusion du flux de lumière.

Le guide de lumière présente une ou plusieurs surfaces d'entrée au travers desquelles ladite source de lumière 41 émet un flux de lumière. L'au moins une surface d'entrée peut correspondre à une face latérale du guide 42. Alternativement, ou de manière complémentaire, l'au moins une surface d'entrée peut correspondre à une partie de la surface supérieure et/ou à une partie de la surface inférieure du guide 42.

En outre, le guide de lumière 42 présente au moins une surface de sortie au travers de la laquelle ressort la lumière provenant de ladite au moins une source de lumière 41. Dans l'invention, ladite au moins une surface de sortie est disposée au moins en regard des éléments photosensibles 31. Ladite au moins une surface de sortie correspond notamment à la surface supérieure et/ou à la surface inférieure du guide 42. Ladite au moins une surface de sortie peut en outre correspondre à au moins une des surfaces latérales du guide de lumière. Ladite surface de sortie est notamment une surface continue. Lorsque la surface de sortie n'est pas disposée en regard des éléments photosensibles 31, elle est préférentiellement en regard d'un organe de réflexion 43 du générateur de lumière 40, d'un dioptre ou d'un réflecteur du détecteur 10, permettant son acheminement ou son renvoi vers les éléments photosensibles 31, afin de maximiser l'éclairage des éléments photosensibles 31.

Le guide de lumière 42 est notamment un guide diffusant, permettant de distribuer le flux lumineux de manière sensiblement constante en tout point de l'au moins une surface de sortie du guide 42.

À cet effet, le guide de lumière 42 peut comprendre notamment une texturation diffusante, un ensemble de microbulles et/ou un ensemble de tâches telles que décrites en relation avec le support 22. Ladite texturation diffusante et ledit ensemble de tâches sont disposées au moins au niveau l'au moins une surface de sortie du guide 42. En particulier elles sont disposées au moins au niveau de la surface de sortie en regard des éléments photosensibles 31 et optionnellement en outre en regard d'au moins un organe de réflexion 43, notamment le réflecteur inférieur 44.

Les différents modes de réalisation en relation avec l'aspect diffusant du guide 42 sont bien entendus combinables entre eux.

Le guide de lumière 42 est notamment transparent ou translucide et a notamment une composition choisie parmi celles énoncées pour le support 22.

Le guide de lumière 42 s'étend au moins sur toute la surface de l'élément adjacent supérieur et/ou inférieur. Le guide 42 a notamment une forme de plaque dont la surface supérieure et la surface inférieure sont au moins égales à celles de l'élément disposé au-dessus ou au-dessous. Le guide de lumière 42 peut également se présenter sous la forme d'une multitude de fibres alignées parallèlement entre elles.

En outre, le guide lumière 42 peut comprendre des dimensions supérieures par rapport à l'élément adjacent supérieur et/ou à l'élément adjacent inférieur. Ainsi, le guide de lumière peut comprendre au moins une partie latérale déportée par rapport à la périphérie du substrat 32 du capteur 30 et/ou de l'écran scintillateur 20. Cet aspect de l'invention permet à l'aide d'un dioptre ou d'un réflecteur supérieure 45 et/ou d'un dioptre ou d'un réflecteur inférieur 44 de minimiser, voire d'éviter une fuite de lumière proche de l'au moins une source de lumière 41 et de concentrer la lumière de l'au moins une source de lumière 41 à l'intérieur du guide.

Le guide lumière 42 peut être disposé à différents niveaux à l'intérieur du détecteur 10. Ainsi, le détecteur peut être disposé :
- directement en aval du substrat 32 du capteur 30,
- entre la substance scintillatrice 21 et les éléments photosensibles 31,
- entre la feuille de protection 23 et la substance scintillatrice 21, et
- en amont du support 22 ou de la feuille de protection 23 de l'écran scintillateur 20.

La composition du substrat 32, de la feuille de protection 23 et du support 22 pourra facilement être adaptée en fonction de la position du guide de lumière 42 et de la nécessité de transmission du flux lumineux provenant de l'au moins une source de lumière 41 ou bien de renvoi de ce dernier.

Selon un mode de réalisation de l'invention, le guide de lumière 42 est au contact par sa surface de sortie en regard des éléments photosensibles 31 avec l'élément adjacent. Ledit élément adjacent peut être le substrat 32 du capteur, la substance scintillatrice 21 de l'écran scintillateur, le support 22 de l'écran scintillateur 20 ou la feuille de protection 23 de l'écran scintillateur 20. L'absence d'espace d'air entre le guide de lumière 42 et l'élément adjacent permet une diffusion plus homogène de la lumière en direction des éléments photosensible 31. Le guide de lumière 42 peut être fixé par tout moyen à l'élément adjacent par sa surface de sortie disposée en regard des éléments photosensibles. Il est ainsi obtenu une structure solidaire où le guide de lumière 42 suit toute déformation de l'élément adjacent à sa surface de sortie en regard des éléments photosensibles 31. Cet aspect permet notamment de s'assurer que l'ensemble des éléments photosensibles 31 reçoive une partie du flux émis par l'au moins une source de lumière 41 et que la distance entre chaque élément photosensible 31 et le guide de lumière 42 reste fixe. Le fait que des variations locales d'intensité de lumière puissent subvenir au niveau d'un ou de plusieurs éléments photosensibles 31 n'est pas problématique dans l'invention.

Cette fixation est notamment réalisée par collage. Le collage peut être réalisé sur tout ou partie de la surface de sortie en regard des éléments photosensibles 31. Notamment, le collage peut être réalisé au niveau de la région de ladite surface de sortie en regard de la zone d'image du capteur 30, ou bien en dehors de cette zone d'image et correspondant dès lors à un collage périphérique. La colle utilisée peut notamment présenter un indice de réfraction inférieur à celui de l'élément adjacent, permettant d'obtenir un meilleur guidage de la lumière. Cet aspect est intéressant lorsque la colle est présente au niveau de la région de ladite surface de sortie en regard de la zone d'image du capteur 30.

En outre, le guide de lumière 42 peut également correspondre au substrat 32 du capteur 30, au support 22 ou à la feuille de protection 23 de l'écran scintillateur 20, et également à la substance scintillatrice 21 de l'écran scintillateur 20.

Le générateur de lumière 40 peut également comprendre un diffuseur 49 disposé entre le guide de lumière 42 et les éléments photosensibles 31, notamment au contact de la ou d'une des surfaces de sortie du guide de de lumière 42. L'aspect diffusant du diffuseur 49 est notamment obtenu via les différents modes de réalisation décrit en relation avec le guide de lumière 42, qui s'appliquent mutatis mutandis. Le diffuseur est dans un matériau transparent ou translucide, tel que décrit précédemment.

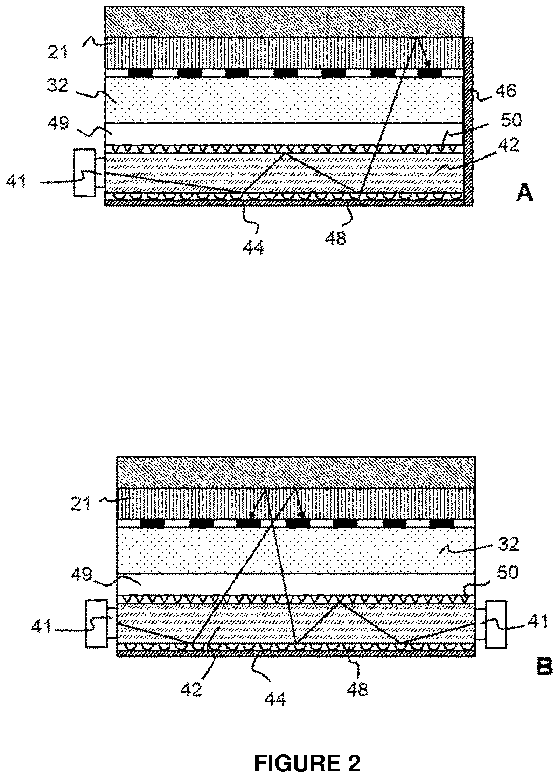

Les figures 1a à 1e permettent d'illustrer cinq configurations selon l'invention de la disposition du guide de lumière 42 par rapport aux autres composants du détecteur 10. Sur ces figures, une seule source de lumière 41 avec son circuit imprimé ont été représentés, pour plus de clarté. D'autres configurations du générateur de lumière 40 sont possibles et illustrées notamment aux figures 2 à 4.

La figure 1a montre une première configuration dans laquelle on retrouve de l'amont vers l'aval du détecteur 10 : le support 22, la substance scintillatrice, les éléments photosensibles 31, le substrat 32 du capteur 30, le guide de lumière 41 et un réflecteur inférieur 44. Ainsi, dans cette configuration, le guide de lumière 41 est disposé du côté de la face arrière du détecteur 10. Dans cette configuration, la surface de sortie du guide de lumière 42 correspond au moins à sa surface supérieure. Ainsi, le flash de lumière émis par le la source de lumière 41 pour l'effacement optique des éléments photosensibles 31 quitte le guide 42 par sa surface supérieure, traverse le substrat 32, passe entre les éléments photosensibles 31 et se réfléchit sur des faces situées en amont du capteur photosensible 30 avant d'atteindre les éléments photosensibles 31 par leur face amont. Plus précisément, le flash de lumière peut se réfléchir à l'interface entre le support 22 et la substance scintillatrice et/ou à l'interface entre l'écran scintillateur 20 et le capteur photosensible 30 sur des faces où se produit une rupture d'indice optique. De façon à se propager le long du guide de lumière 42, le flash de lumière se réfléchit contre le réflecteur inférieur 44 et peut également se réfléchir sur la face arrière des éléments photosensibles 31 et/ou à l'interface entre le guide de lumière 42 et le substrat 32 du capteur 30. Ainsi la surface arrière des éléments photosensibles 31 peut être apte à réfléchir la lumière visible. Il est ainsi augmenté la possibilité que la lumière de la source de lumière 41 atteigne l'ensemble des éléments photosensibles 31. Le support 22 est notamment ici dans un matériau opaque.

Dans la configuration représentée, le substrat 32 doit être transparent ou translucide de manière à laisser passer la lumière émise par le générateur 40.

Dans cette configuration, le guide de lumière 42 est notamment au contact, et en particulier collé, par sa surface supérieure avec le substrat 32 du capteur 30.

Le réflecteur inférieur 44 est apte ici à renvoyer la lumière sortant par la face inférieure du guide 42 vers le guide 42 et notamment en direction de sa face supérieure afin d'atteindre les couches supérieures et les éléments photosensibles 31.

La figure 1b représente une deuxième configuration dans laquelle on retrouve de l'amont vers l'aval du détecteur 10 : le support 22, la substance scintillatrice 21, le guide de lumière 42 et le capteur 30. Dans cette configuration, la surface de sortie du guide de lumière 42 correspond au moins à sa surface inférieure et à sa surface supérieure, cette dernière permettant aux photons en provenance de l'écran scintillateur 20 d'atteindre les éléments photosensibles 31. Ainsi, le flash de lumière émis par la source de lumière 41 arrive directement aux éléments photosensibles 31 depuis la surface inférieure du guide de lumière 42. Il n'y a donc pas besoin d'organe de réflexion 43 dans cette configuration. De façon à se propager le long du guide de lumière 42, le flash de lumière peut se réfléchir à l'interface entre le guide de lumière 42 et l'écran scintillateur 20 et/ou à l'interface entre la substance scintillatrice 21 et le support 22. Le support 22 est notamment ici dans un matériau opaque. Le guide de lumière 42 peut notamment être au contact des éléments photosensibles 31. Avantageusement dans cette configuration, la diffusion de la lumière jusqu'aux éléments photosensibles 31 n'est pas gênée par les ces derniers.

La figure 1c représente une troisième configuration dans laquelle on retrouve de l'amont vers l'aval du détecteur 10 : la feuille de protection 23, le guide de lumière 42, la substance scintillatrice 31 et le capteur 30. Dans cette configuration, la surface de sortie du guide de lumière correspond a moins à sa surface inférieure. Ici, le flash de lumière émis par le générateur 40 quitte le guide 42 sa surface inférieure, traverse la substance scintillatrice 21 et arrive aux éléments photosensible 31. A l'image de la deuxième configuration représentée en figure 1b, l'organe de réflexion 43 n'est pas nécessaire dans cette configuration. De façon à se propager le long du guide de lumière 42, le flash de lumière peut se réfléchir à l'interface entre le guide de lumière 42 et la feuille de protection 23. Avantageusement dans cette configuration également, la diffusion de la lumière jusqu'aux éléments photosensibles 31 n'est pas gênée par ces derniers.

Notamment, le guide de lumière 42 est notamment au contact, et en particulier collé, par sa surface inférieure avec la substance scintillatrice 21.

Les figures 1d et 1e représentent respectivement une quatrième et une cinquième configuration où le guide de lumière 42 n'est pas un élément rapporté au détecteur 10. Dans la quatrième configuration, c'est le substrat 32 du capteur 30 fait office de guide de lumière 42. Dans la cinquième configuration, c'est le support 22 qui fait office de guide de lumière 42. Ces configurations sont avantageuses en ce qu'elles permettent une réduction de l'encombrement du générateur de lumière 40 et des coûts de production du détecteur 10.

Dans la quatrième configuration, on retrouve de l'amont vers l'aval du détecteur 10 : le support 22, la substance scintillatrice 21, le capteur 30 et un réflecteur inférieure 44. Ici, la surface de sortie du guide 42 correspond au moins à la surface supérieure du substrat 32. Dans cette configuration, le flash de lumière émis par la source de lumière 41 quitte le substrat 32 par sa surface supérieure, passe entre les éléments photosensibles 31 et se réfléchit sur des faces situées en amont du capteur photosensible 30 avant d'atteindre les éléments photosensibles 31 par leur face amont, à l'image de la première configuration. Plus précisément, le flash de lumière peut se réfléchir à l'interface entre le support 22 et la substance scintillatrice et/ou à l'interface entre l'écran scintillateur 20 et le capteur photosensible 30 sur des faces où se produit une rupture d'indice optique. Le support 22 est notamment dans un matériau opaque. De façon à se propager le long du guide de lumière 42, le flash de lumière se réfléchit contre le réflecteur inférieur 44 et peut également se réfléchir sur la face arrière des éléments photosensibles 31. Ainsi, dans cette configuration, la surface arrière des éléments photosensible 31 peut également être apte à réfléchir la lumière visible.

Dans la configuration représentée, le substrat 32 doit être transparent ou translucide de manière à laisser passer la lumière émise par la source de lumière 41 du générateur 40.

Dans la cinquième configuration, on retrouve de l'amont vers l'aval du détecteur 10 : un réflecteur supérieur 45, le support 22, la substance scintillatrice 21 et le capteur 30. Ici, la surface de sortie du guide 42 correspond au moins à la surface inférieure du support 22. Ainsi, le flash de lumière émis par la source de lumière 41 arrive directement aux éléments photosensibles 31 via la surface inférieure de la substance scintillatrice 21. De façon à se propager le long du guide de lumière 42, le flash de lumière peut se réfléchir à l'interface entre le réflecteur supérieur 45 et le support 22. Avantageusement dans cette configuration, la diffusion de la lumière jusqu'au élément photosensible 31 n'est pas gênée par les ces derniers.

Dans la configuration représentée, le support 22 doit être transparent ou translucide de manière à laisser passer la lumière émise par la source de lumière 41 du générateur 40.

On se tournera à présent vers les figures 2 à 4 qui représentent trois configurations du générateur de lumière 40 en relation avec la première configuration représentée en figure 1a. Néanmoins l'homme du métier saura aisément adapter les configurations du générateur de lumière 40 représentées ici aux configurations illustrées aux figures 1b à 1e relative au guide lumière 42. Pour chaque configuration du guide de lumière 42 illustrée aux figures 2 à 4 est représenté un mode de réalisation comprenant une seule source de lumière 41 disposée en périphérie d'une face latérale du guide de lumière 42 (A) ainsi qu'un mode de réalisation comprenant deux sources de lumière 41 disposées en périphérie de deux faces latérales opposées du guide de lumière 42 (B).

Les figures 2A et 2B illustrent une première configuration dans laquelle la ou les sources de lumières 41 sont au contact d'une face latérale du guide 42 et le guide de lumière 42 présente une surface supérieure et inférieure de même dimension que celle du substrat 32 et de l'écran scintillateur 20. Le trajet d'un photon du flux de lumière depuis une source de lumière 41 est représenté.

Plus précisément, dans la figure 2A le générateur de lumière 40 comprend :
- une ou plusieurs sources de lumières disposées au contact d'une seule face latérales du guide de lumière,
- un guide de lumière s'étendant sous toute la surface du substrat 32 du capteur 30 et comprenant une texturation sous la forme d'une pluralité de points de sérigraphie 48 au niveau de sa surface inférieure,
- un diffuseur 49 disposé entre le substrat 32 et le guide de lumière 42 et comprenant une texturation sous la forme d'une indentation 50 au niveau de sa surface inférieure,
- un réflecteur inférieur 44 et
- un réflecteur latéral 46 disposé contre la face latérale du guide 42 opposée à celle de l'au moins une source de lumière 41, et s'étendant le long d'une face latérale du diffuseur 49, du substrat 32 et de la substance scintillatrice 21.

A la figure 2B cette configuration est reprise à l'exception du réflecteur latéral 46 qui est remplacé par une ou plusieurs autres sources de lumière 41.

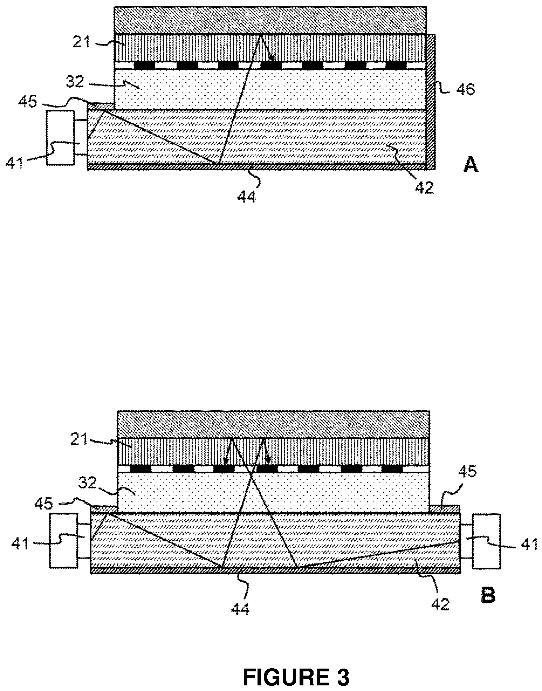

Les figures 3A et 3B illustrent une deuxième configuration dans laquelle la ou les sources de lumières 41 sont au contact d'une face latérale du guide 42 et le guide de lumière présente une surface supérieure et inférieure de dimensions supérieures à celle du substrat 32 et de l'écran scintillateur 20. Le guide de lumière présente ainsi une ou deux parties latérales déportées. Le trajet d'un photon du flux de lumière depuis une source de lumière 41 est représenté.

Le mode de réalisation représenté à la figure 3A comprend :
- une ou plusieurs sources de lumières disposées au contact d'une seule face latérale de la partie latérale déportée du guide de lumière 42,
- un guide de lumière 42 présentant une surface supérieure et inférieure de dimensions supérieures à celles du substrat 32, de sorte à comprendre une partie latérale déportée,
- un réflecteur inférieur 44,
- un réflecteur supérieur 45 disposé contre la surface supérieure de la partie latérale déportée du guide de lumière 42 et
- un réflecteur latéral 46 disposé contre la face latérale du guide 42 opposée à celle de l'au moins une source de lumière 41, et s'étendant le long d'une face latérale du substrat 32 et de la substance scintillatrice 21.

A la figure 3B cette configuration est reprise à l'exception du réflecteur latéral 46 qui est remplacé par une ou plusieurs autres sources de lumière 41 disposées au niveau d'une deuxième partie latérale déportée opposée à la première et recouverte par un deuxième réflecteur latéral 46.

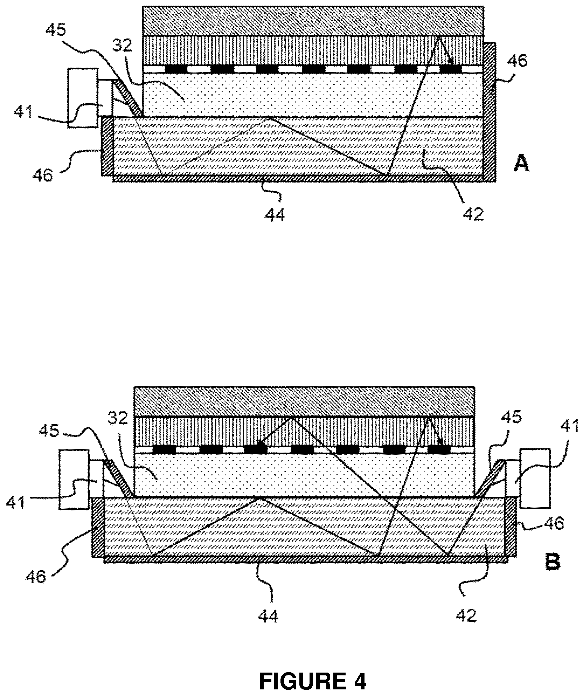

Les figures 4A et 4B illustrent une troisième configuration dans laquelle la ou les sources de lumières 41 sont disposées au-dessus d'une face latérale du guide 42, où le guide de lumière 42 présente une surface supérieure et inférieure de dimension supérieure à celle du substrat 32 et de l'écran scintillateur 20, où un réflecteur latéral 46 est disposé au niveau de chaque face latérale du guide 42 et où un réflecteur supérieure 45 oblique est présent de sorte à diriger la lumière provenant desdites sources 41 vers l'intérieur du guide 42. Le trajet d'un photon du flux de lumière depuis une source de lumière 41 est représenté.

## Revendications

1. Détecteur numérique (10) à l'état solide d'un rayonnement incident (100), le détecteur (10) comprenant un capteur photosensible (30) et un générateur de lumière (40), le capteur photosensible (30) comprenant des éléments photosensibles (31) organisés en matrice, le générateur de lumière (40) étant destiné à effacer optiquement les éléments photosensibles (31), où le générateur de lumière (40) comprend :
- un guide de lumière (42) comprenant une face avant tournée vers l'avant du détecteur (10), une face arrière opposée à la face avant et au moins une face latérale s'étendant entre la face avant et la face arrière, et
- au moins une source de lumière (41) disposée en périphérie latérale du guide de lumière (42) et où le générateur de lumière (40) est configuré pour que ladite au moins une source de lumière (41) injecte de la lumière au travers d'au moins une des faces du guide de lumière (42),
ledit guide de lumière (42) étant configuré pour répartir la lumière provenant de ladite au moins une source de lumière (41) sur toute la matrice d'éléments photosensibles (31).

2. Détecteur numérique selon la revendication 1, dans lequel le guide de lumière (42) comprend une texturation diffusante afin d'obtenir une répartition uniforme de la lumière au niveau de la matrice d'éléments photosensibles (31).

3. Détecteur de lumière selon la revendication 1 ou 2, dans lequel le guide de lumière (42) comprend un ensemble de microbulles judicieusement disposées en son sein afin d'obtenir une répartition uniforme de la lumière au niveau de la matrice d'éléments photosensibles (31).

4. Détecteur numérique selon l'une des revendications 1 à 3, dans lequel le capteur photosensible comprend un substrat (32) transparent ou translucide sur lequel est disposée la matrice d'éléments photosensibles (31) et où la face avant du guide de lumière (42) est disposée contre ledit substrat (32).

5. Détecteur numérique selon la revendication 4, dans lequel le guide de lumière (42) est fixé au substrat (32) du capteur photosensible (30), de préférence par collage.

6. Détecteur numérique selon l'une des revendications 1 à 3, dans lequel le capteur photosensible comprend un substrat (32) transparent ou translucide sur lequel est disposée la matrice d'éléments photosensibles (31) et où ledit substrat (32) forme le guide de lumière (42).

7. Détecteur numérique selon l'une des revendications 1 à 6, dans lequel ladite au moins une source de lumière (41) est disposée en regard d'une au moins des faces latérales du guide de lumière (42).

8. Détecteur numérique selon l'une des revendications 1 à 7, dans lequel le générateur de lumière (40) comprend au moins un organe de réflexion (43) apte à dévier la lumière provenant de ladite au moins une source de lumière (41) vers le guide de lumière (42).

9. Détecteur numérique selon la revendication 7, dans lequel l'organe de réflexion (43) comprend au moins un dioptre et/ou au moins un réflecteur (44, 45, 46) disposé en regard d'une des faces du guide de lumière (42).

10. Détecteur numérique selon l'une des revendications 1 à 9, dans lequel le guide de lumière (42) comprenant au moins deux faces latérales opposées entre elles, dans lequel le générateur de lumière (40) comprend au moins un réflecteur latéral (46) disposé contre au moins une des faces latérales du guide de lumière (42), de préférence ladite au moins une source de lumière (41) est disposée en regard de la face latérale opposée à celle du réflecteur latéral (46).

11. Détecteur numérique (10) selon l'une des revendications 1 à 10, dans lequel la matrice d'éléments photosensibles (31) est disposée sur un substrat (32) transparent ou translucide, où le guide de lumière (42) est disposé en aval dudit substrat (32) et comprend une partie latérale déportée par rapport à celle dudit substrat (32), où le générateur de lumière (40) comprend au moins un réflecteur supérieur (45) s'étendant contre tout ou partie de la face supérieure de ladite au moins une partie latérale déportée du guide de lumière (42), et où ladite au moins une source de lumière (41) est disposée en périphérie de ladite au moins une partie latérale déportée du guide de lumière (42).
